(19) Europäisches Patentamt
European Patent Office
Office européen des brevets

(11) Veröffentlichungsnummer : **0 036 160**
**B1**

(12) # EUROPÄISCHE PATENTSCHRIFT

(45) Veröffentlichungstag der Patentschrift : **11.05.83**

(51) Int. Cl.³ : **C 07 D311/58**

(21) Anmeldenummer : **81101714.4**

(22) Anmeldetag : **09.03.81**

(54) **Verfahren zur Herstellung von 2-Hydroxyalkylchromanen.**

(30) Priorität : **19.03.80 DE 3010504**

(43) Veröffentlichungstag der Anmeldung : **23.09.81 Patentblatt 81/38**

(45) Bekanntmachung des Hinweises auf die Patenterteilung : **11.05.83 Patentblatt 83/19**

(84) Benannte Vertragsstaaten : **BE CH DE FR GB IT LI NL**

(56) Entgegenhaltungen :
**CH A 469 699**
**CH A 565 782**
**CH A 602 700**
**DE A 2 364 141**
**DE A 2 364 165**

(73) Patentinhaber : **BASF Aktiengesellschaft**
**Carl-Bosch-Strasse 38**
**D-6700 Ludwigshafen (DE)**

(72) Erfinder : **Horner, Michael, Dr.**
**Im Ritterbueschel 9**
**D-6730 Neustadt (DE)**
Erfinder : **Nissen, Axel, Dr.**
**Panoramastrasse 51**
**D-6906 Leimen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**EP 0 036 160 B1**

Verfahren zur Herstellung von 2-Hydroxyalkylchromanen

Die vorliegende Erfindung betrifft ein neues Verfahren zur Herstellung von 2-Hydroxyalkylchromanen der allgemeinen Formel I

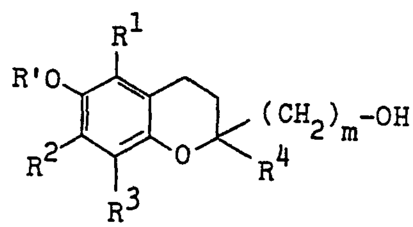

(I)

in welcher die Substituenten und der Index folgende Bedeutung haben :

$R^1$ bis $R^4$H ; $C_1$-$C_8$-Alkyl

R'H ; hydrolytisch oder hydrogenolytisch als R'OH oder R'—COOH abspaltbarer organischer Rest

m 1, 2 oder 3.

Verbindungen dieses Typs sind aus der DE-OS 23 64 141 bekannt. Nach dem dort beschriebenen Verfahren stellt man sie her, indem man ein Hydrochinon (II)

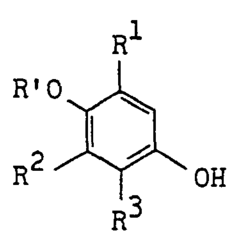

(II)

in Gegenwart eines sauren Katalysators mit einem Alkendiolmonoether oder -monoester (III')

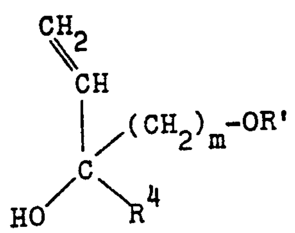

(III')

umsetzt. Als saure Katalysatoren werden hierbei Lewis-Säuren, Mineralsäuren oder Mischungen von Lewis-Säuren und Mineralsäuren, und zwar besonders eine Mischung aus Salzsäure und Zinkchlorid, empfohlen.

Dieses Verfahren ist jedoch umständlich und unwirtschaftlich, da es die Herstellung von Monoethern bzw. Monoestern der den Verbindungen III' zugrundeliegenden Diole IIIa

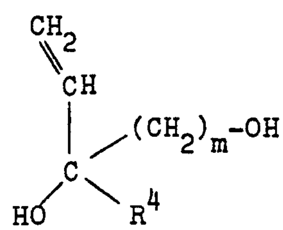

(IIIa)

voraussetzt.

« Ferner war es aus der CH-PS 565 782 bekannt, Chromane durch Umsetzung eines Hydrochinons mit einem 1,3-Dien herzustellen. Da sich die Diene bei der Kondensation zum Chromansystem wie bifunktionelle Verbindungen verhalten und beide Funktionen der Annellierung dienen, kann man hierbei

lediglich solche Chromanderivate erhalten, die am sauerstoffhaltigen Ring Alkylgruppen als Substituenten tragen. Derartige Chromanderivate sind jedoch für weitere Synthesen praktisch nicht geeignet.

Ähnlich verhält es sich mit dem Verfahren der CH-PS 602 700, denn auch hier erhält man nur alkyl- oder alkenylsubstituierte Chromanderivate. »

Aufgabe der Erfindung war es daher, die 2-Hydroxyalkylchromane I unmittelbar aus den Hydrochinonen II und den freien Alkendiolen IIIa oder anderen als Bausteinen geeigneten Alkendiolen herzustellen.

Es wurde gefunden, daß man 2-Hydroxyalkylchromane der allgemeinen Formel I

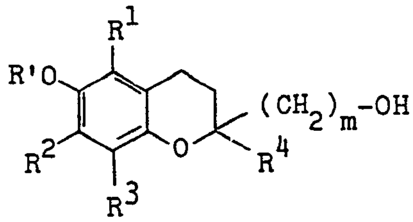

(I)

in der die Substituenten und der Index folgende Bedeutung haben

$R^1$ bis $R^4$ H ; $C_1$-$C_8$-Alkyl

R'H ; hydrolytisch oder hydrogenolytisch als R'—OH oder R'—COOH abspaltbarer organischer Rest

m 1, 2 oder 3

auf einfache und wirtschaftliche Weise erhält wenn man ein Hydrochinon II

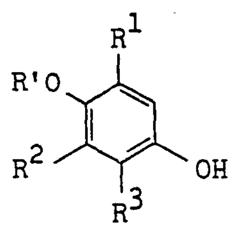

(II)

a) mit einem Alkendiol IIIa

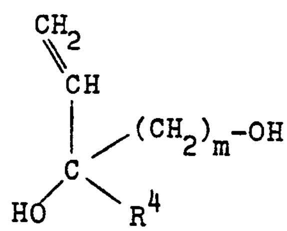

(IIIa)

oder

b) mit einem Alkendiol IIIb

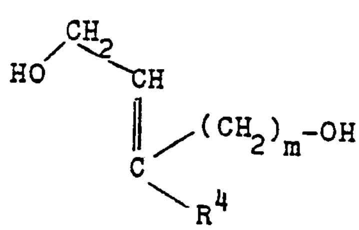

(IIIb)

oder

c) mit einem Alkendiol IIIc

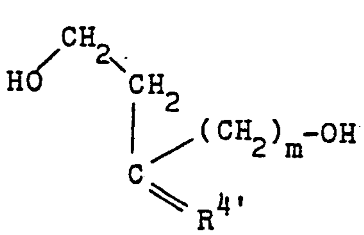

(IIIc)

wobei $R^{4'}$ ein Rest ist, der bei der Kondensation mit II in den Rest $R^4$ übergeht bei ( – 50) bis 120 °C in Gegenwart von 10 bis 300 mol.-%, bezogen auf die Menge von II, einer Lewissäure oder eines Adduktes aus einer Lewissäure mit einer Lewisbase, deren Basizität geringer ist als die der Verbindungen II, in einem aprotischen Lösungsmittel umsetzt, dessen Donizität (gemäß Definition in « Angewandte Chemie », Band 82 (1972), S. 858) höchstens 20 ist.

Die als Ausgangsverbindungen dienenden Hydrochinone II sind bekannt oder nach bekannten Methoden erhältlich. Im Hinblick auf die Eigenschaften der Verfahrensprodukte und auch auf die Zugänglichkeit des Hydrochinons II hat das Trimethylhydrochinon die größte Bedeutung.

Sofern die Reste $R^1$ und/oder $R^2$ Wasserstoff bedeuten, empfiehlt es sich, die zwischen ihnen liegende OH-Gruppe durch Veretherung oder Veresterung mittels eines Alkohols R'—OH bzw. einer Säure R'—COOH in an sich bekannter Weise zu schützen, damit man nicht verschiedene Cycloadditionsprodukte von II mit den Alkendiolen III erhält. Prinzipiell eignet sich als Rest R' in der Schutzgruppe jeder beliebige organische Rest, der seinerseits, wie sich allerdings von selbst versteht, keine Substituenten trägt, die zu störenden Nebenreaktionen Anlaß geben. Aus praktischen Gründen verwendet man jedoch Schutzgruppen, in denen

R' ein $C_1$-$C_4$-Alkylrest oder ein Benzylrest ist. Nach beendeter Synthese wird die Schutzgruppe dann wieder hydrolytisch oder durch Hydrierung in bekannter Weise wieder abgespalten. Auch für den Fall, daß $R^1$ und/oder $R^2 \neq H$ sind, kann sich das Arbeiten mit der geschützten OH-Gruppe empfehlen, besonders dann, wenn das Hydrochinon II bereits von seiner Synthese her in geschützter Form vorliegt.

Die Ausgangsverbindungen III (IIIa bis IIIc) sind bekannt oder nach bekannten Verfahren erhältlich. Allgemein werden diejenigen Verbindungen III bevorzugt, in denen $R^4$ für Wasserstoff oder insbesondere für die Methylgruppe steht.

Als Alkendiole IIIa kommen demgemäß vor allem

But-3-en-1,2-diol
Pent-4-en-1,3-diol
2-Methylbut-3-en-1,2-diol
3-Methylpent-4-en-1,3-diol

in Betracht. Man erhält diese Verbindungen durch partielle Hydrierung der entsprechenden Alkindiole, durch Umsetzung von Carbonylverbindungen $R^4$—CO—$(CH_2)_m$—OH mit Vinyl-MgCl oder durch Umlagerung der Verbindungen IIIb.

Als Alkendiole IIIb eigenen sich vornehmlich :

But-2-en-1,4-diol
Pent-2-en-1,5-diol
2-Methylbut-2-en-1,4-diol
3-Methylpent-2-en-1,5-diol.

Diese Verbindungen können durch partielle Hydrierung der entsprechenden Alkindiole oder durch oxidative Addition von Essigsäure an 1,3-Diene und anschließende Verseifung sowie durch Isomerisierung der Verbindungen IIIc hergestellt werden.

Die bevorzugten Alkendiole IIIc

2-Methylenbutan-1,4-diol
3-Methylenpentan-1,5-diol

sind durch Isomerisierung der Verbindungen IIIb erhältlich.

Als Lewissäuren eigenen sich beispielsweise Borhalogenide wie $BF_3$ und $BCl_3$, Aluminiumhalogenide wie $AlCl_3$ und $AlBr_3$, Zinntetrahalogenide wie $SnCl_4$ und $SnBr_4$ und Antimontrihalogenide wie $SbF_3$ und $SbCl_3$.

Weiterhin eignen sich die Addukte von Lewissäuren mit solchen Lewisbasen, deren Basenstärke geringer ist als die der Verbindung II. Derartige Verbindungen sind u. a. Addukte aus den vorgenannten Lewissäuren einerseits und Ethern wie Dimethylether, Diethylether und Tetrahydrofuran, Estern wie Ethylacetat und organischen Nitroverbindungen wie Nitromethan, Nitropropan, Nitrobenzol und den Nitrotoluolen andererseits. Unter den Lewissäuren bzw. Lewissäure/Lewisbase-Addukten werden $BF_3$, $AlCl_3$ und $BF_3$-Diethyletherat bevorzugt.

Die Menge der Lewissäuren bzw. der Addukte beträgt allgemein 10 bis 300 mol.-%, bezogen auf die Menge des Hydrochinons, jedoch empfehlen sich in der Regel Mengen zwischen 50 und 200, besonders zwischen 80 und 120 mol.-%.

Als inerte Lösungsmittel eignen sich allgemein aprotische Flüssigkeiten, deren Donizität höchstens 20 ist. Der Begriff der Donizität ist in der Arbeit von V. Gutmann, Angewandte Chemie, Band 82 (1972), S. 858, definiert. Er ist ein Ausdruck für die Affinität zu Lewissäuren und bedeutet für den Fall der vorliegenen Maßgabe, daß die Lösungsmittel mit den Lewissäuren keine stabilen Anlagerungskomplexe bilden sollen. Geeignete Lösungsmittel sind demgemäß z. B. Dichlormethan, 1,2-Dichloräthan, Chloroform, Benzol, Toluol, Chlorbenzol, Nitromethan, Nitrobenzol, Benzonitril, Acetonitril, Cyclohexan und Mischungen dieser Lösungsmittel.

Unter den genannten Lösungsmitteln sind Gemische von Toluol oder Dichlormethan mit Nitromethan besonders gut geeignet. Die Funktion der Lösungsmittel ist es, daß mindestens ein Teil der eingesetzten Reaktionspartner in homogener Lösung gehalten wird. Die Menge der Lösungsmittel kann in weiten Grenzen schwanken, beträgt im allgemeinen jedoch 1 bis 10 kg pro kg II.

Die Reaktionstemperaturen liegen vorzugsweise zwischen (– 50) und 100 °C, vor allem zwischen (– 40) und 60 °C. Da die Reaktion unter Umständen sehr heftig verlaufen kann, was zu einer vermehrten Bildung unerwünschter Produkte führen kann, empfiehlt es sich, bei niedrigen Temperaturen zu beginnen und die Temperatur dann allmählich zu erhöhen. Eine zu heftige Reaktion kann auch durch allmähliche Zugabe der Reaktionspartner vermieden werden.

Da die Reaktion bei Normaldruck ausgeführt werden kann, besteht in aller Regel kein Grund, sie unter erniedrigtem oder erhöhtem Druck vorzunehmen.

Sonstige verfahrenstechnische Besonderheiten sind nicht zu beachten, d. h. man kann die Umsetzung nach den üblichen präparativen Techniken vornehmen, indem man z. B. eine Lösung oder Suspension des Hydrochinons II vorlegt und hierzu gleichzeitig allmählich eine Lösung eines Alkendiols III sowie die Lewissäure oder eine Lösung der Lewissäure oder des Lewissäure-Adduktes zugibt.

Die Aufarbeitung der Reaktionsgemische kann nach den üblichen Methoden erfolgen, wie sie für Friedel-Crafts-Reaktionen bekannt sind, etwa indem man das Gemisch mit Wasser und gegebenenfalls zusätzlich mit Ether versetzt und die Lösungsmittel von der getrockneten organischen Phase abdestilliert. Das so erhältliche Rohprodukt, welches I in etwa 50 bis 90 %iger Ausbeute enthält, kann gewünschtenfalls durch Umkristallisation, z. B. aus Aceton/Wasser, gereinigt werden. Meistens kristallisieren die Verfahrensprodukte bereits in sehr reiner Form aus der nach der Hydrolyse erhaltenen organischen Phase aus, so daß sich eine weitere Reinigung erübrigt.

Die 2-Hydroxyalkylchromane I sind wichtige Zwischenprodukte für die Herstellung von Vitamin E und damit verwandten Antioxidantien, die ihrerseits als Stabilisatoren für organische Massen wie Fetten, Ölen und Kunststoffen dienen.

## Beispiele 1 bis 14

Herstellung von 2,5,7,8-Tetramethyl-6-hydroxy-2-(2-hydroxyethyl)-chroman (Ia)

### Beispiel 1

Herstellung von Ia aus 3-Methylpent-2-en-1,5-diol

Eine Lösung aus 112,5 g (0,84 mol) $AlCl_3$, 750 ml Methylenchlorid und 100 ml Nitromethan wurde bei (– 10) °C zunächst mit 128 g (0,845 mol) Trimethylhydrochinon und danach mit 98 g (0,845 mol) 3-Methylpent-2-en-1,5-diol versetzt. Diese Lösung wurde anschließend 10 Stunden bei Raumtemperatur gerührt.

Zur Aufarbeitung wurde die so erhaltene braune Reaktionslösung unter Kühlung mit 150 ml Wasser hydrolysiert, wobei das Verfahrensprodukt als kristalliner Niederschlag ausfiel. Dieser Niederschlag wurde zweimal mit Wasser gewaschen und getrocknet. Die Ausbeute an Ia betrug 83 %. Weitere 8 % wurden aus der Flüssigphase des Hydrolysegemisches gewonnen, indem diese eingeengt und der Rückstand mit Toluol extrahiert wurde. Smp. von Ia : 118 bis 120 °C.

### Beispiel 2

Herstellung von Ia aus 3-Methylpent-4-en-1,3-diol

Eine Lösung aus 21,9 g (0,164 mol) $AlCl_3$, 150 ml Methylenchlorid und 20 ml Nitromethan wurde bei (– 10) °C mit 25 g (0,16 mol) Trimethylhydrochinon und anschließend innerhalb von 25 Minunten mit 19,1 g (0,164 mol) 3-Methylpent-4-en-1,3-diol versetzt und danach 12 Stunden bei Raumtemperatur gerührt. Die Aufarbeitung des Reaktionsgemisches analog Beispiel 1 lieferte das Chromanderivat Ia in 78 %iger Ausbeute.

### Beispiel 3

Herstellung von Ia aus 3-Methylenpentan-1,5-diol

Auf die in Beispiel 2 beschriebene Weise, jedoch mit Ethylenchlorid anstelle des Methylenchlorids wurde das oben genannte Diol in 51 %iger Ausbeute, bezogen auf das eingesetzte Hydrochinon, in das Chromanderivat Ia überführt.

Beispiele 4 bis 14

Herstellung von Ia aus 3-Methylpent-2-en-1,5-diol unter Verwendung verschiedener Lewissäuren

Auf die in Beispiel 1 angegebene Weise, jedoch mit jeweils gleich molaren Mengen anderer Lewissäuren als $AlCl_3$ wurde Ia mit den aus der folgenden Tabelle ersichtlichen Ergebnissen hergestellt :

| Beispiel Nr. | Lewissäure | Umsatz, bezogen auf TMH* % | Ausbeute, bezogen auf eingesetztes TMH* % |
|---|---|---|---|
| 1 | $AlCl_3$ | 99 | 91 |
| 4 | $BF_3$ | 91 | 79 |
| 5 | $BF_3.OEt_2$ | 70 | 63 |
| 6 | $AlBr_3$ | 95 | 85 |
| 7 | $ZnCl_2$ | 78 | 39 |
| 8 | $ZnBr_2$ | 84 | 68 |
| 9 | $ZnJ_2$ | 95 | 23 |
| 10 | $SnCl_4$ | 93 | 60 |
| 11 | $SnBr_4$ | 96 | 66 |
| 12 | $BCl_3$ | 58 | 39 |
| 13 | $SbCl_3$ | 95 | 69 |
| 14 | $SbF_3$ | 55 | 27 |

*) Trimethylhydrochinon

Beispiel 15

Herstellung von 2,5,7,8-Tetramethyl-6-hydroxy-2-hydroxymethyl-chroman (Ib)

Die Herstellung dieser Verbindung erfolgte analog Beispiel 1, jedoch mit 2-Methylbut-2-en-1,4-diol anstelle des Pentendiols. Die Ausbeute an Ib betrug 10 % ; Smp. 123 bis 125 °C (nach Umkristallisation aus Diethylether/Petrolether).

**Anspruch**

Verfahren zur Herstellung von 2-Hydroxyalkylchromanen der allgemeinen Formel I

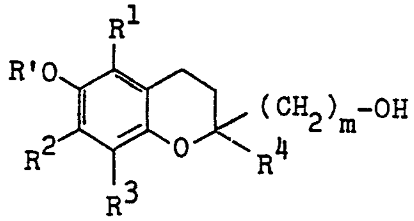

(I)

in der die Substituenten und der Index folgende Bedeutung haben

$R^1$ bis $R^4$ H ; $C_1$-$C_8$-Alkyl

R' H ; hydrolytisch oder hydrogenolytisch als R'—OH oder R'—COOH abspaltbarer organischer Rest

m 1, 2 oder 3

dadurch gekennzeichnet, daß man ein Hydrochinon II

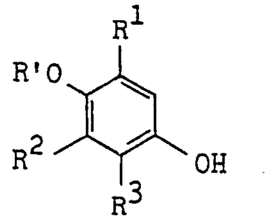

(II)

a) mit einem Alkendiol IIIa

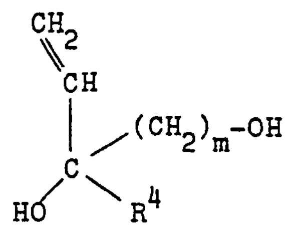

(IIIa)

oder

b) mit einem Alkendiol IIIb

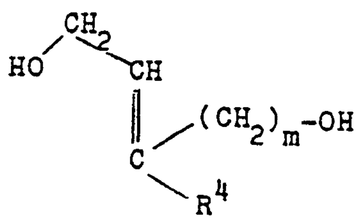

(IIIb)

oder

c) mit einem Alkendiol IIIc

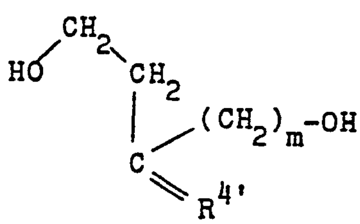

(IIIc)

wobei $R^{4'}$ ein Rest ist, der bei der Kondensation mit II in den Rest⁴ übergeht bei (– 50) bis 120 °C in Gegenwart von 10 bis 300 mol.-%, bezogen auf die Menge von II, einer Lewissäure oder eines Adduktes aus einer Lewissäure mit einer Lewisbase, deren Basizität geringer ist als die der Verbindung II, in einem aprotischen Lösungsmittel umsetzt, dessen Donizität (gemäß Definition in « Angewandte Chemie », Band 82 (1972), S. 858) höchstens 20 ist.

**Claim**

A process for the preparation of a 2-hydroxyalkylchroman of the general formula I

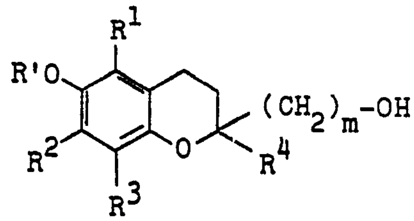

(I)

where

$R^1$, $R^2$, $R^3$ and $R^4$ are each H or $C_1$-$C_8$-alkyl,

R' is H or an organic radical which can be split off hydrolytically or hydrogenolytically as R'—OH or R'—COOH, and

m is 1, 2 or 3,

wherein a hydroquinone II

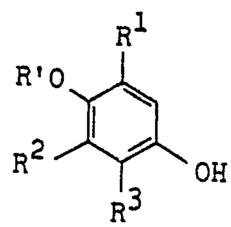

(II)

is reacted

a) with an alkenediol IIIa

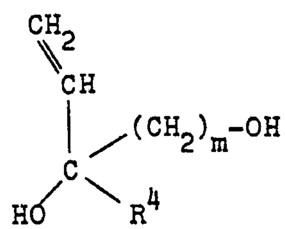

(IIIa)

or

b) with an alkenediol IIIb

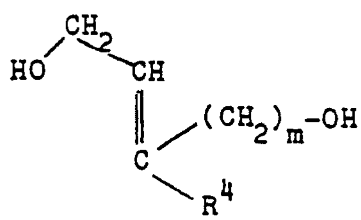

(IIIb)

or

c) with an alkenediol IIIc

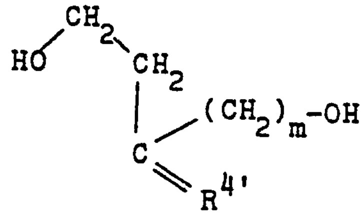

(IIIc)

where $R^{4'}$ is a radical which during the condensation with II is converted to the radical $R^4$, at from − 50 °C to 120 °C, in an aprotic solvent having a donicity (as defined in « Angewandte Chemie »,

82, 858 (1972) of at most 20, in the presence of from 10 to 300 mole.-%, based on II, of a Lewis acid or of an adduct of a Lewis acid with a Lewis base which has a lower basicity than compound II.

**Revendication**

Procédé pour la préparation de 2-hydroxyalcoylchromanes de formule générale I

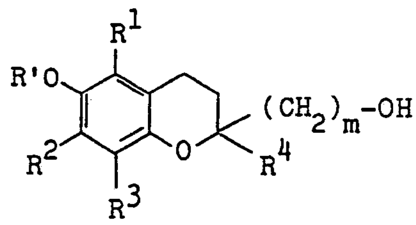

(I)

dans laquelle les substituants et l'indice ont les significations suivantes

$R^1$ à $R^4$ H ; alcoyle en $C_1$ à $C_8$

R'H ; radical organique séparable par hydrolyse ou hydrogénolyse sous la forme R'—OH ou R'—COOH

m 1, 2 ou 3,

caractérisé en ce qu'on fait réagir une hydroquinone II

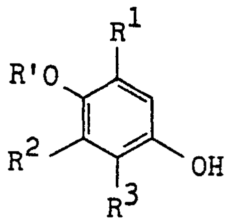

(II)

a) avec un alcène-diol IIIa

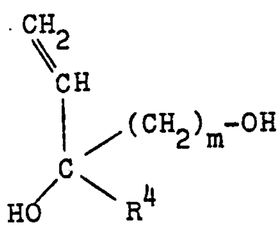

(IIIa)

ou

b) avec un alcène-diol IIIb

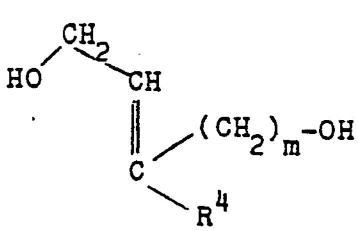

(IIIb)

ou

c) avec un alcène-diol IIIc

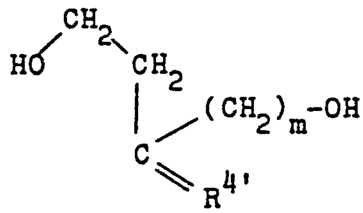

(IIIc)

($R^{4'}$ étant un radical qui se transforme en le radical $R^4$ lors de la condensation avec II) à une température de (− 50) à 120 °C, en présence de 10 à 300 mol.-%, par rapport à la quantité de II, d'un acide de Lewis ou d'un produit d'addition d'un acide de Lewis avec une base de Lewis, dont la basicité est plus faible que celle du composé II, dans un solvant aprotique dont la donicité (suivant la définition donnée dans « Angewandte Chemie », vol. 82 (1972), p. 858) est au maximum de 20.